# EUROPEAN PATENT SPECIFICATION

(11) **EP 3 824 911 B1**
(45) Date of publication and mention of the grant of the patent: **08.11.2023**
(21) Application number: 20208749.0
(22) Date of filing: 19.11.2020
(51) Int. Cl.: A61L 2/26, E05F 15/63

(54) **MACHINE FOR TREATING OBJECTS WITH CLOSING DEVICE**
MASCHINE ZUR BEHANDLUNG VON GEGENSTÄNDEN MIT VERSCHLUSSVORRICHTUNG
MACHINE DE TRAITEMENT D'OBJETS AVEC DISPOSITIF DE FERMETURE

(30) Priority: 25.11.2019 IT 201900022080
(43) Date of publication of application: 26.05.2021
(73) Proprietor: Steelco S.p.A., 31039 Riese Pio X (IT)
(72) Inventor: Capovilla, Ivone, 31037 Loria (TV) (IT)
(74) Representative: Petraz, Gilberto Luigi

(56) References cited:
- WO-A1-2018/087320
- CN-U- 201 621 825
- CN-U- 207 598 090
- US-A1- 2014 311 042

## Description

### FIELD OF THE INVENTION

The invention concerns a machine for treating objects, in particular a cleaning/disinfection machine provided with a closing device.

### BACKGROUND OF THE INVENTION

Machines for treating objects, in particular cleaning/disinfection apparatuses, are used in structures such as, for example, clinics, hospitals, care centers for the elderly or other similar structures, where it is necessary to clean and/or disinfect objects or instruments that come into contact with human organic material.

To introduce such objects into a cleaning/disinfection apparatus, they must be handled by an operator who has to position them in the cleaning compartment of the apparatus.

Given that human organic material can be potentially infectious, it is necessary to provide devices that, during the operation to load the instruments, the external parts of the device, or those accessible from outside the device, must not be handled by the operator. In fact, hands and gloves, if used, may have come into contact with the organic material, and therefore, for example in order to open the door of the cleaning compartment, if the hands touch it, they deposit the organic material.

Therefore, to avoid contact between the operator and the door, the apparatuses are provided with closing devices in which the door is driven by drive means that allow automatic movement and thus prevent direct contact with the door.

The drive means are normally associated with a drive unit configured to drive the door.

In the state of the art, drive units typically comprise a transmission mean, typically a chain associated with a system of toothed gears. The transmission unit is associated with the door in such a way as to transmit the motion generated by the drive means to it, thus allowing the compartment to open/close.

Using a chain mechanism requires the use of specific elements associated with it, for example gears, chain tensioners and others, which create a transmission unit that comprises a large number of elements and parts, interconnected and/or cooperating with each other.

One disadvantage of known closing devices is that the replacement of a broken or worn element may require a long and laborious temporary disassembly of the unit in order to reach the element concerned.

Furthermore, the chain, and the gears with which it is associated, require regular and appropriate adjustment so that the tension of the chain is optimal over time.

One disadvantage of transmission units using a chain is that they need constant routine maintenance.

It is also known that in chain closing devices, only the closing movement of the door is entrusted to the activation of a motorized mean. The movement of opening the door typically occurs due to gravity, therefore with the motorized mean not active.

Given that, on the side of the door facing the cleaning compartment, objects to be cleaned can be placed on suitable housings, the weight of said objects affects and influences the opening movement of the door.

This weight can therefore lead to an abrupt opening movement which can generate strong stresses on the transmission unit, reducing its time of use and increasing the need for maintenance operations.

Furthermore, an abrupt opening movement of the door can cause vibrations which can lead to the accidental fall of such objects positioned on it, as well as splashes of liquid or other that could possibly be present.

Another disadvantage is that closing devices made by means of said transmission units are usually bulky, which is reflected in the size of the apparatus in which the closing device is installed.

Devices for moving doors or suchlike are known, which use crank-type transmission units in the mining and automotive sectors, as described, respectively, in US2014/0311042 A1 and in CN 201621825 U. WO 2018/087320 A1 discloses a machine for treating objects comprising a door opened and closed with a crank-type transmission unit.

There is therefore a need to perfect a closing device for doors of machines for treating objects which can overcome at least one of the disadvantages of the state of the art.

The purpose of the invention is to provide a machine for treating objects, for example a cleaning and disinfection apparatus equipped with an access door, provided with a device for closing said door according to the invention.

The Applicant has devised, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the present invention or variants to the main inventive idea.

In accordance with the above purposes, the invention concerns a a machine for treating objects, in particular a machine for cleaning and disinfecting sanitary items, but not only, which comprises a frame, a treatment chamber provided with an aperture that can be opened or closed by a closing device.

According to the invention, the closing device is equipped with a flap door, configured to rotate with respect to a corresponding hinging axis, a transmission member and a drive mean.

Such transmission member comprises:
- a first crank, provided with one end associated with the drive mean, and with an opposite end cooperating with
- a second crank provided with one end associated with a rotation pintle, which defines the hinging axis on which the door rotates, and an opposite free end associated with the first crank
- a transmission strut, which advantageously achieves the association between the two cranks.

The axis of rotation of the second crank is stably associated with the part of the door and constitutes the rotation pintle thereof.

According to the invention, the rotation of the first crank at least partly operates on the second crank, about its axis of rotation, in a counter-clockwise direction or in a clockwise direction wherein such counter-clockwise direction and such clockwise direction coincide respectively with a corresponding opening rotation and closing rotation of the door.

The closing device supplied by the invention therefore provides to use a transmission member which significantly reduces the number of parts and components necessary to move the door.

The transmission member is therefore not only compact and simple to make, but also introduces better control of the movement of the door, both in the closing step and, in particular, in the opening step.

The movement of the door in the steps described above is in fact achieved in both cases by activating the drive mean. This means that even the opening of the door, which in known devices is typically obtained by gravity, is instead a movement controlled by the drive mean.

According to the invention, the movements of the second crank can have opposite directions to each other and are obtained with a rotation of the first crank which has the same direction, that is, a single or univocal direction.

In this way, it is possible to use a simpler and economical drive mean compared to drive means with a double direction of rotation.

The machine for treating objects, in particular for cleaning and disinfecting, comprises a frame, a treatment chamber provided with an aperture, which can be opened or closed by the closing device.

According to some embodiments, the machine for treating objects can be manufactured reducing its bulk without sacrificing the capacity of the treatment chamber.

This advantage can allow to more easily adapt the treatment machine to the spaces and layouts of the premises in which it is to be disposed, which typically have to house a plurality of tools and machines.

### BRIEF DESCRIPTION OF THE DRAWINGS

These and other aspects, characteristics and advantages of the present invention will become apparent from the following description of some embodiments, given as a non-restrictive example with reference to the attached drawings wherein:
- fig. 1 shows a machine for treating objects in accordance with embodiments described here;
- figs. 2 and 3 show a closing device in accordance with embodiments described here in two different positions;
- figs. 4 and 5 show a detail of the closing device in accordance with embodiments described here in two different positions;

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We will now refer in detail to the possible embodiments of the invention, of which one or more examples are shown in the attached drawings. Each example is supplied by way of illustration of the invention and shall not be understood as a limitation thereof. For example, the characteristics shown or described insomuch as they are part of one embodiment can be adopted on, or in association with, other embodiments to produce other embodiments. It is understood that the present invention shall include all such modifications and variants.

Before describing these embodiments, we must also clarify that the present description is not limited in its application to details of the construction and disposition of the components as described in the following description using the attached drawings. The present description can provide other embodiments and can be obtained or executed in various other ways. We must also clarify that the phraseology and terminology used here is for the purposes of description only, and cannot be considered as limitative.

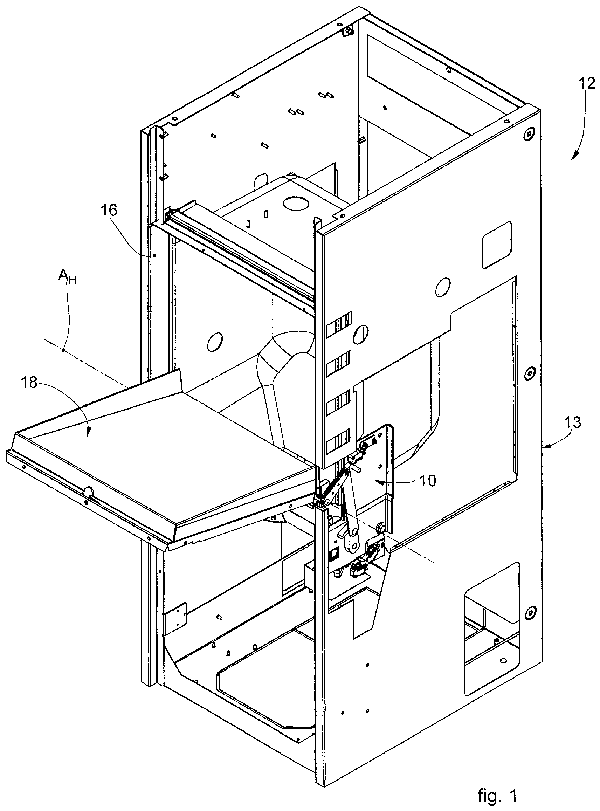

With reference to the attached drawings, some embodiments described here concern a closing device 10 and a machine 12 for treating objects, in particular a machine for cleaning and disinfecting or other, which comprises a frame 13, a treatment chamber 14 provided with an aperture 16 that can be opened or closed by the closing device 10 (fig. 1).

The closing device 10 comprises a flap door 18, configured to rotate with respect to a corresponding hinging axis Ah, a transmission member 20 and a drive mean 22 (figs. 1, 2 and 3).

In correspondence with the access aperture 16, along the edge, there can be positioned a packing, not visible in the drawings, for sealing the treatment chamber 14, suitable to cooperate with the door 18 once the latter has been closed correctly.

According to the invention, the transmission member 20 comprises:
- a first crank 24, provided with one end associated with the drive mean 22, and with an opposite end cooperating with
- a second crank 26 provided with one end associated with a rotation pintle 28, which defines the hinging axis Ah on which said door 18 rotates, and an opposite free end associated with the first crank
- a transmission strut 30 which advantageously achieves the association between the first crank 24 and the second crank 26.

According to the invention, the rotation of the first crank 24 at least partly moves the second crank 26, about its axis of rotation, in an anti-clockwise direction R1 or in a clockwise direction R2 wherein such anticlockwise direction R1 and such clockwise direction R2 respectively coincide with a corresponding opening rotation and closing rotation of the door 18.

The hinging axis Ah is positioned in correspondence with one end of the door 18.

In some embodiments, the second crank 26 is directly connected to the rotation pintle 28.

According to some embodiments, the drive mean 22 can be configured to move the first crank 24 with a rotation R3 about an axis of rotation Ar.

According to the invention, the movement R1 and the movement R2 of the second crank 26, which have opposite directions with respect to each other, are obtained with a rotation R3 of the first crank 24 which has a single direction.

Compared to the state of the art, for example chain closing mechanisms, which require the use of drive means that provide a rotation in two directions, the invention can allow to use drive means provided to supply a rotation in a single direction which are simpler and cheaper.

Furthermore, since the opening and closing movement of the door correspond respectively to movement R1 and movement R2, this means that the movement of the door always occurs in a controlled manner and accompanied by the drive mean 22.

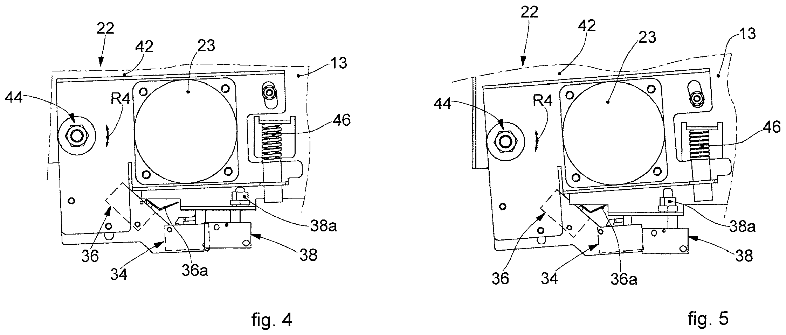

In accordance with some embodiments, the drive mean 22 can comprise a motor 23 which can be associated with a motor support 42 (fig. 4).

Some embodiments provide that the drive mean 22 can be associable with the frame 13 of a treatment machine 12 by means of an attachment element 44 which allows at least a partial rotation movement R4 thereof about an axis of rotation defined by the attachment element 44 as above.

The drive mean 22 can be further associated, in a position substantially opposite the attachment element 44, with an elastic mean 46 suitably configured to contrast the possible movement R4 of the drive mean 22, as better described below.

According to some embodiments, the closing device 10 can comprise command elements 32, 34, configured to control the start or stop of the drive mean 22 as above.

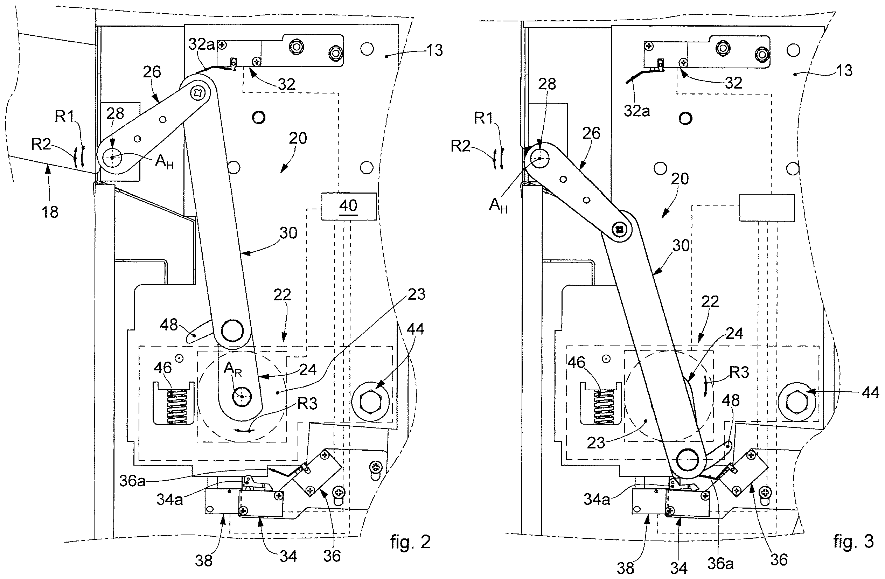

In other preferred embodiments, in addition to the command elements 32, 34, the closing device 10 also comprises additional command elements 36, 38 (figs. 2 and 3).

The following description and corresponding drawings concern a preferred embodiment of the invention which comprises command elements 32, 34, 36, 38.

In possible embodiments, the command elements 32, 34, 36, 38 can be connected to a control unit 40 configured to receive the signals from the command elements 32, 34, 36, 38 as above, possibly process them and send them to the drive mean 22 and then control the start or stop thereof.

In particular, as better described below, the command elements 32, 34, 36 can detect specific positions of the transmission member 20.

The command element 38 can detect the position of the drive mean 22, with respect to the frame 13.

According to some embodiments, during the rotation motion R3 of 360°, the transmission member 20 can assume a plurality of discrete positions, in at least one of which the transmission member 20 cooperates with, in particular contacts, one or more command elements 32, 34, 36.

According to some embodiments, the command elements 32, 34, 36 are provided with respective contact elements 32a, 34a, 36a, configured to intentionally and at least temporarily cooperate with, in particular contact, the transmission member 20.

In accordance with some embodiments, the command element 38 is provided with a contact element 38a configured to intentionally and at least temporarily cooperate with, in particular contact, the drive mean 22.

The contact elements 32a, 34a, 36a, 38a, when contacted, can generate the opening or closing of a circuit that activates or deactivates the function of the corresponding command element 32, 34, 36, 38.

The contact elements 32a, 34a, 36a, 38a can be, for example, foils made of electrically conductive material, or be elements that can be engaged by pressure.

In further embodiments, the contact elements 32a, 34a, 36a, 38a can be sensors, for example optical such as photocells or of another type, in any case suitable to detect the presence of a body.

With the help of figs. 2-5, we will now describe some of the positions that the transmission member 20, during the movement R3, and the drive mean 22 can assume, and the corresponding association with the command elements 32, 34, 36, 38.

It is understood that the choice of depicting these positions is solely for the purposes of providing an example to support understanding of the invention, therefore they are not to be considered as the only possible positions.

With reference to fig. 2, this shows a closing device 10 in which the transmission member 20 is positioned so that the door 18 is open. In this position, the transmission member 20 is configured to contact the contact element 32a of the command element 32.

According to some embodiments, the contact element 32a of the command element 32 can be contacted by the transmission strut 30, in particular the contact can occur in correspondence with the end of the transmission strut 30 pivoted to the second crank 26.

In accordance with some embodiments, the contact between the end of the transmission strut 30 and the contact element 32a provides a signal that the door 18 has reached the open position and therefore induces the drive mean 22 to stop.

According to some embodiments, the closing device 10 can be provided with a safety system that blocks the closing movement R2 of the door, preventing the motor 23 from starting or commanding it to stop if already started.

This safety system can be provided by the command element 38.

According to some embodiments, the drive mean 22 can be driven only in the event that the contact element 38a is contacted, in particular with the drive mean 22 (fig. 4).

Cases may occur in which a force contrary to the closing movement R2 of the door 18 is applied to the door and this force can act on the drive mean 22 since the drive mean 22 and the door 18 are connected by the transmission member 20.

The drive mean 22, since it is configured to be partly mobile with respect to the frame 13, can be disassociated from the contact element 38a when the applied force contrary to the closing movement R2 of the door is such as to overcome the resistance of the elastic mean 46 (fig. 5).

Consequently, the command element 38 provides a signal to block the functioning of the motor 23.

The situations in which the safety system can advantageously be activated can be, for example, an excessively heavy object weighing on the door or an obstacle interposed between the door and the frame of the apparatus, such as for example an object protruding from the treatment chamber or the hand of a careless operator.

In those cases where an excessively heavy object weighs on the door, that is, the weight bearing on the door is greater than the weight that the drive mean can lift according to its technical specifications, the safety system prevents the drive mean from straining and being damaged.

In those cases where, on the other hand, an object or a hand is interposed between the door and the frame, the safety system prevents not only possible damage to the drive mean but also damage to the person or object.

With reference to fig. 3, a closing device 10 can be seen in which the transmission member 20 is positioned so that the door 18 is closed. In this position, the transmission member 20 is configured to simultaneously contact the contact element 34a and 36a of the command element 34 and 36 respectively.

According to some embodiments, the contact elements 34a and 36a of the command element 34 and 36 can be contacted by the first crank 24, in particular the contact occurs with the free end of the first crank 24.

In accordance with some embodiments, the free end of the first crank 24 can have a cantilevered extension 48, which has a surface configured to contact the command elements 34, 36, in particular the contact elements 34a and 36a, which has a shape substantially approximating a sphere or a portion of a sphere. In particular, the surface configured to contact the contact elements 34a and 36a can develop as an arc portion of a circumference that has a center substantially coincident with the axis of rotation Ar of the first crank 24.

According to some embodiments, the extension 48 is configured in such a way as to simultaneously contact, at least temporarily, the contact elements 34a and 36a.

In accordance with some embodiments, the contact between the first crank 24 and the contact element 34a provides the signal that the door 18 has reached the closed position and therefore induces the motor 23 to stop.

According to other embodiments, the closing device 10 can comprise an additional command element (not shown) which can contact the door 18, when the latter is closed, to provide a further signal that the closing has occurred.

When the door is in correspondence with the closing position, that is, before the first crank 24 and the contact element 34a come into contact, the drive mean 22 may have to overcome a resistance generated by the insertion of the profile of the door with the aperture 16 of the treatment machine. This resistance generates a force contrary to the closing movement of the door which can be sufficient to allow the drive mean 22 to overcome the resistance of the elastic mean 46. This eventuality would then trigger the safety system, preventing a total closing of the door 18.

For this reason, the first crank 24 is configured to contact the contact element 36a of the command element 36 which is configured to provide a signal that excludes the function of the command element 38. As a consequence, even in the event the drive mean 22 were to overcome the resistance of the elastic mean 46, and therefore disassociate itself from the command element 38, there would not be a blocking of the motor 23, therefore the closing of the door can be completed.

Therefore, the rotation movement R3 of the first crank 24 from the position of door open of fig. 2 to the position of door closed of fig. 3 can imply that the first crank 24 contacts, intentionally temporarily, the contact element 36a before the contact element 34a.

In this way, the movement R3 of the first crank 24 can be allowed to close the door 18 until there is contact with the contact element 34a of the command element 34.

According to some embodiments, the first crank 24 can be configured to contact the command element 36 when the door 18 is in a position in which, advantageously, it is not possible for an operator to insert a hand. As a consequence, the command element 38 can be deactivated only when the risk of a possible injury to the person is excluded.

According to some embodiments, the treatment machine 12 can be provided with suitable devices able to provide a desired signal of start of the drive mean 22 in order to command the opening or closing of the door 18 (not shown). These devices, not shown, can be mechanically or optically activated switches, such as for example photocells, or proximity sensors or other.

These switches can be advantageously positioned so that they can even be reached by an operator whose hands are occupied during the loading step of the treatment machine.

It is clear that modifications and/or additions of parts may be made to the closing device and to the treatment machine as described heretofore, without departing from the field and scope of the present invention as defined by the claims.

It is also clear that, although the present invention has been described with reference to some specific examples, a person of skill in the art shall certainly be able to achieve many other equivalent forms of closing device and treatment machine, having the characteristics as set forth in the claims and hence all coming within the field of protection defined thereby.

In the following claims, the sole purpose of the references in brackets is to facilitate reading: they must not be considered as restrictive factors with regard to the field of protection claimed in the specific claims.

## Claims

1. Machine (12) for treating objects, in particular for cleaning and disinfecting, **characterized in that** it comprises a frame (13), a treatment chamber (14) provided with an aperture (16) and a closing device (10) for closing said aperture (16), said closing device (10) comprising a flap door (18) configured to rotate with respect to a corresponding hinging axis (Ah), a transmission member (20) and a drive mean (22), wherein said transmission member (20) comprises:
- a first crank (24), provided with one end associated with the drive mean (22), and with an opposite end cooperating with
- a second crank (26) provided with one end associated with a rotation pintle (28), which defines the hinging axis (Ah) on which said door (18) rotates, and an opposite free end associated with the first crank (24)
- a transmission strut (30) which advantageously achieves the association between the first crank (24) and the second crank (26),
said closing device being configured so that the rotation of the first crank (24) at least partly moves the second crank (26), about its axis of rotation, in a counter-clockwise direction (R1) or in a clockwise direction (R2) wherein said counter-clockwise direction (R1) and said clockwise direction (R2) coincide respectively with a corresponding opening rotation and closing rotation of the door (18), wherein the movement (R1) and the movement (R2) of the second crank (26), which have opposite directions to each other, are obtained with a rotation (R3) of the first crank (24) which has a single direction.

2. Machine (12) as in claim 1, **characterized in that** it comprises command elements (32, 34, 36, 38) configured to control the start or stop of said drive mean (22).

3. Machine (12) as in claim 2, **characterized in that** during the rotation motion (R3) of 360°, said transmission member (20) can assume a plurality of discrete positions, at least in one of which said transmission member (20) contacts one or more command elements (32, 34, 36).

4. Machine (12) as in claim 2 or 3, **characterized in that** said command elements (32, 34, 36) detect specific positions of the transmission member (20) **and in that** said command element (38) detects the position of the drive mean (22).

5. Machine (12) as in any of claims 2 to 4 **characterized in that** the free end of the first crank (24) has a cantilevered extension (48), which has a surface configured to contact the command elements (34, 36).

6. Machine (12) as in any claim hereinbefore, **characterized in that** said drive mean (22) can be associated with the frame (13) by means of an attachment element (44) which allows at least a partial rotation movement (R4) thereof about an axis of rotation defined by said attachment element (44).

7. Machine (12) as in claim 6, **characterized in that** said drive mean (22) is also associated, in a position substantially opposite the attachment element (44), with an elastic mean (46) suitably configured to contrast the possible movement (R4) of the drive mean (22).

8. Machine (12) as in claim 6 or 7, dependent on any of claims 2-5, **characterized in that** the drive mean (22), being configured to be partly mobile with respect to the frame (13), dissociates itself from the command element (38), when a force applied against the closing movement (R2) of the door is such as to overcome the resistance of the elastic mean (46).

## Patentansprüche

1. Gerät (12) zur Behandlung von Objekten, insbesondere zum Reinigen und Desinfizieren, **dadurch gekennzeichnet, dass** es einen Rahmen (13), eine Behandlungskammer (14), die mit einer Öffnung (16) und einer Schließvorrichtung (10) zum Verschließen der Öffnung (16) versehen ist, wobei die Schließvorrichtung (10) eine Klapptür (18), die zum Rotieren hinsichtlich eines dazugehörigen Scharniers (Ah) ausgestaltet ist, ein Übertragungselement (20) und ein Antriebsmittel (22) umfasst, wobei das Übertragungselement (20) umfasst:
- eine erste Kurbel (24), die mit einem Ende, das mit dem Antriebsmittel (22) in Verbindung steht, und mit einem gegenüberliegenden Ende versehen ist, das zusammenarbeitet mit
- einer zweiten Kurbel (26), die mit einem Ende, das mit einem Drehbolzen (28) in Verbindung steht, welcher das Scharnier (Ah) definiert, auf dem die Tür (18) rotiert, sowie einem gegenüberliegenden freien Ende versehen ist, das mit der ersten Kurbel (24) zusammenarbeitet,
- eine Übertragungsstrebe (30), welche vorteilhafterweise die Verbindung zwischen der ersten Kurbel (24) und der zweiten Kurbel (26) liefert,
wobei die Schließvorrichtung derart ausgestaltet ist, dass die Rotation der ersten Kurbel (24) wenigstens teilweise die zweite Kurbel (26) bewegt, und zwar über ihre Rotationsachse in Richtung gegen den Uhrzeigersinn (R1) oder in Richtung des Uhrzeigers (R2), wobei die Richtung gegen den Uhrzeigersinn (R1) und die Richtung im Uhrzeigersinn (R2) jeweils mit einer entsprechenden Öffnungsrotation und Schließrotation der Tür (18) übereinstimmen, wobei die Bewegung (R1) und die Bewegung (R2) der zweiten Kurbel (26) die gegensätzliche Richtungen zueinander haben, mit einer Rotation (R3) der ersten Kurbel (24) erhalten werden, welche eine einzige Richtung hat.

2. Gerät (12) gemäß Anspruch 1, **dadurch gekennzeichnet, dass** es Befehlselemente (32, 34, 36, 38) umfasst, die zum Steuern des Starts und Stopps der Antriebsmittel (22) ausgestaltet sind.

3. Gerät (12) gemäß Anspruch 2, **dadurch gekennzeichnet, dass** während der Rotationsbewegung (R3) von 360° das Übertragungselement (20) eine Vielzahl an diskreten Positionen annehmen kann, wobei in wenigstens einer davon das Übertragungselement (20) ein oder mehrere der Befehlselemente (32, 34, 36) kontaktiert.

4. Gerät (12) gemäß Anspruch 2 oder 3, **dadurch gekennzeichnet, dass** die Befehlselemente (32, 34, 36) spezielle Positionen des Übertragungselements (20) detektieren, und dass das Befehlselement (38) die Position des Antriebsmittels (22) detektiert.

5. Gerät (12) gemäß einem der Ansprüche 2 bis 4, **dadurch gekennzeichnet, dass** das freie Ende der ersten Kurbel (24) eine Auskragung (48) aufweist, welche eine Oberfläche hat, die zum Kontakt der Befehlselemente (34, 36) ausgestaltet ist.

6. Gerät (12) gemäß einem der vorangehenden Ansprüche, **dadurch gekennzeichnet, dass** das Antriebselement (22) mit dem Rahmen (13) mittels eines Vorsatzelementes (44) in Verbindung stehen kann, welches wenigstens eine teilweise Drehbewegung (R4) davon über eine Rotationsachse ermöglicht, die durch das Vorsatzelement (44) definiert ist.

7. Gerät (12) gemäß Anspruch 6, **dadurch gekennzeichnet, dass** das Antriebselement (22) ebenso mit einem elastischen Mittel (46) in Verbindung steht, und zwar in einer Position, die im wesentliche gegenüber dem Vorsatzelement (44) liegt, wobei das elastische Mittel (46) zweckmäßigerweise derart ausgestaltet ist, dass es einer möglichen Bewegung (R4) des Antriebsmittels (22) entgegenwirkt.

8. Gerät (12) gemäß Anspruch 6 oder 7, abhängig von irgendeinem der Ansprüche 2 bis 5, **dadurch gekennzeichnet, dass** das Antriebsmittel (22), welches derart ausgestaltet ist, dass es hinsichtlich des Rahmens (13) teilweise mobil ist, sich selbst von dem Befehlselement (38) löst, wenn eine gegen die Schließbewegung (R2) der Tür angewendete Kraft derart ist, dass sie den Widerstand des elastischen Elements (46) überwindet.

## Revendications

1. Machine (12) de traitement d'objets, en particulier de nettoyage et de désinfection, **caractérisée en ce qu'**elle comprend un cadre (13), une chambre de traitement (14) pourvue d'une ouverture (16) et un dispositif de fermeture (10) pour fermer ladite ouverture (16), ledit dispositif de fermeture (10) comprenant une porte à volet (18) configurée pour tourner par rapport à un axe d'articulation correspondant (Ah), un élément de transmission (20) et des moyens d'entraînement (22), dans lequel ledit élément de transmission (20) comprend :
- une première manivelle (24), pourvue d'une extrémité associée au moyen d'entraînement (22), et d'une extrémité opposée coopérant avec
- une seconde manivelle (26) pourvue d'une extrémité associée à un pivot de rotation (28), qui définit l'axe d'articulation (Ah) sur lequel ladite porte (18) tourne, et d'une extrémité libre opposée associée à la première manivelle (24)
- une entretoise de transmission (30) qui réalise avantageusement l'association entre la première manivelle (24) et la seconde manivelle (26),
ledit dispositif de fermeture étant configuré de sorte que la rotation de la première manivelle (24) déplace au moins partiellement la seconde manivelle (26), autour de son axe de rotation, dans une direction dans le sens inverse des aiguilles d'une montre (R1) ou dans une direction dans le sens des aiguilles d'une montre (R2) dans lequel ladite direction dans le sens inverse des aiguilles d'une montre (R1) et ladite direction dans le sens des aiguilles d'une montre (R2) coïncident respectivement avec une rotation d'ouverture et de fermeture correspondante de la porte (18), dans lequel le mouvement (R1) et le mouvement (R2) de la seconde manivelle (26), qui présentent des directions opposées l'une à l'autre, sont obtenus avec une rotation (R3) de la première manivelle (24) qui présente une direction unique.

2. Machine (12) selon la revendication 1, **caractérisée en ce qu'**elle comprend des éléments de commande (32, 34, 36, 38) configurés pour commander le démarrage ou l'arrêt desdits moyens d'entraînement (22).

3. Machine (12) selon la revendication 2, **caractérisée en ce que** pendant le mouvement de rotation (R3) de 360°, ledit élément de transmission (20) peut prendre une pluralité de positions distinctes, au moins dans l'une desquelles ledit élément de transmission (20) vient en contact avec un ou plusieurs éléments de commande (32, 34, 36).

4. Machine (12) selon la revendication 2 ou 3, **caractérisée en ce que** lesdits éléments de commande (32, 34, 36) détectent des positions spécifiques de l'élément de transmission (20) et **en ce que** ledit élément de commande (38) détecte la position des moyens d'entraînement (22).

5. Machine (12) selon l'une quelconque des revendications 2 à 4, **caractérisée en ce que** l'extrémité libre de la première manivelle (24) présente une extension en porte-à-faux (48), qui présente une surface configurée pour venir en contact avec les éléments de commande (34, 36).

6. Machine (12) selon l'une quelconque des revendications précédentes, **caractérisée en ce que** lesdits moyens d'entraînement (22) peuvent être associés au cadre (13) au moyen d'un élément de fixation (44) qui permet au moins un mouvement de rotation partiel (R4) de ceux-ci autour d'un axe de rotation défini par ledit élément de fixation (44).

7. Machine (12) selon la revendication 6, **caractérisée en ce que** lesdits moyens d'entraînement (22) sont également associés, dans une position sensiblement opposée à l'élément de fixation (44), à des moyens élastique (46) configurés de manière appropriée pour contrer le mouvement possible (R4) des moyens d'entraînement (22).

8. Machine (12) selon la revendication 6 ou 7, dépendant de l'une quelconque des revendications 2 à 5, **caractérisée en ce que** les moyens d'entraînement (22), étant configurés pour être partiellement mobiles par rapport au cadre (13), se dissocient de l'élément de commande (38), lorsqu'une force appliquée à l'encontre du mouvement de fermeture (R2) de la porte est telle qu'elle surmonte la résistance des moyens élastique (46).
